## Europäisches Patentamt

## European Patent Office

## Office européen des brevets

(11) Veröffentlichungsnummer: **0 067 929**
**B1**

(12) **EUROPÄISCHE PATENTSCHRIFT**

(45) Veröffentlichungstag der Patentschrift:
**12.12.84**

(21) Anmeldenummer: **82102153.2**

(22) Anmeldetag: **17.03.82**

(51) Int. Cl.³: **A 61 F 1/00**

(54) Sehnen- und/oder Bänderersatz.

(30) Priorität: **24.06.81 CH 4171/81**

(43) Veröffentlichungstag der Anmeldung:
**29.12.82 Patentblatt 82/52**

(45) Bekanntmachung des Hinweises auf die Patenterteilung:
**12.12.84 Patentblatt 84/50**

(84) Benannte Vertragsstaaten:
**AT DE FR GB IT NL**

(56) Entgegenhaltungen:
**EP - A - 0 041 111**
**DE - A - 2 513 284**
**DE - A - 2 717 615**
**US - A - 3 805 300**
**US - A - 3 842 441**

(73) Patentinhaber: **GEBRÜDER SULZER
AKTIENGESELLSCHAFT, Zürcherstrasse 9,
CH-8401 Winterthur (CH)**

(72) Erfinder: **Frey, Otto, Walrütistrasse 56,
CH-8400 Winterthur (CH)**
Erfinder: **Semlitsch, Manfred, Dr., Endlikerstrasse 86,
CH-8400 Winterthur (CH)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf (DE)**

## Beschreibung

Die Erfindung betrifft einen Sehnen- und/oder Bänderersatz. Ein Sehnen- und/oder Bänderersatz ist beispielsweise aus der US-A-3 805 300 bekannt; das »tragende Gerüst« dieser Konstruktion besteht aus einer nicht dehnbaren — jedoch biegsamen — gezwirnten, gewobenen oder geflochtenen Schnur oder einem Metalldraht, die am freien Ende und im eigentlichen Sehnenbereich mit einem biokompatiblen Kunststoff beschichtet sind, um ein unkontroliertes Einwachsen von Gewebe in den Sehnenersatz zu verhindern. Das andere Ende der Schnur ist durch ebenfalls aus Kunststoff bestehende Umhüllungen gegen Abrieb geschützt. Der komplizierte Aufbau einer natürlichen Sehne, bei der dünne Einzelfasern oder -fäden zu einem Strang zusammengefaßt sind, dessen einzelne Fäden bei einer Bewegung um die Längsachse des Stranges eine Drehung ausführen und daher relativ zueinander verschiebbar sind, wird mit dieser Konstruktion nur ungenügend nachgeahmt.

Die Erfindung geht aus von einem Sehnen- und/oder Bänderersatz, wie er in der nicht vorveröffentlichten EP-A-0 041 111 offenbart ist und zum Stand der Technik gemäß Art 54 (3) EPÜ gehört. In dieser Druckschrift wird ein Sehnen- und/oder Bänderersatz vorgeschlagen, der aus einzelnen Metallfäden besteht die zumindestens in einem Strang mit vom Kern nach außen zunehmender Elastizität zusammengefaßt sind, wobei die einzelnen Fäden und der gesamte Strang je von einer losen Umhüllung umschlossen sind; diese Umhüllung besteht vorzugsweise aus einem textilen Geflecht, kann jedoch auch eine Kunststoffummantelung sein. Trotz dieser Umhüllung sind die Metallfäden bei der vorgeschlagenen Sehnenkonstruktion dem chemischen Angriff der Körperflüssigkeiten ausgesetzt. Sie müssen daher nicht nur aus Metallen bzw. Legierungen mit sehr hohen mechanischen Festigkeiten, vor allem einer hohen Zugfestigkeit und einer geringen Dehnbarkeit, sondern auch mit hoher Korrosionsbeständigkeit gegenüber Körperflüssigkeiten gefertigt werden. Darüber hinaus sind die einzelnen Fäden eines solchen Sehnen- und/oder Bänderersatzes einem erheblichen Verschleiß durch Abrieb unterworfen, da sie sich dauernd aufeinander gleitend relativ zueinander verschieben. Ihr Material muß daher zusätzlich noch möglichst abriebfest sein. Die einzelnen Fäden werden daher aus hochlegierten Materialien, wie z. B. CoCrMo- oder CoNiCrMo-Schmiedelegierungen, hergestellt, wobei unter Umständen bei der Auswahl der Legierung nicht für alle drei Forderungen — optimale mechanische Eigenschaften, besonders in punkto Zugfestigkeit und geringe Dehnbarkeit, Korrosionsbeständigkeit und Verschleißfestigkeit — optimale Werte erreicht werden, sondern bei einer oder zwei der Eigenschaften Konzessionen zugunsten der dritten gemacht werden müssen. Aufgabe der Erfindung ist es daher, einen Sehnen- und/oder Bänderersatz auf möglichst wirtschaftliche Weise aus einzelnen Fäden so aufzubauen, daß er den gestellten Forderungen in allen Punkten möglichst weitgehend entspricht. Diese Aufgabe wird erfindungsgemäß gelöst durch einen Sehnen- und Bänderersatz aus einzelnen, in Längsrichtung mindestens annähernd parallel verlaufenden Fäden aus einem körperverträglichen Metall und/oder einer Metallegierung, die zu mindestens einem Strang, bestehend mindestens aus einem Kernfaden und einer Reihe ihn umgebender Mantelfäden, zusammengefaßt sind, wobei jeder einzelne Faden mit einer körperverträglichen, korrosionsbeständigen und abriebfesten festhaftenden Beschichtung aus Metall oder einer Hartstoffschicht überzogen ist.

Mit diesem Vorgehen ist es möglich, das Material des Metallfadens aufgrund allein seiner mechanischen Eigenschaften und/oder nach wirtschaftlichen Gesichtspunkten auszuwählen und beispielsweise die Abriebfestigkeit durch eine geeignete Beschichtung zu erreichen oder — wenn man die erwähnten, bisher zu diesem Zweck verwendeten Materialien anwendet — zu verbessern.

Neben den bereits erwähnten Legierungen können daher für die Sehnen oder Bänder bevorzugt rostfreie Stähle, wie z. B. AISO — 316 L, Titan oder Titanlegierungen eingesetzt werden. Als Beschichtungen eignen sich in erster Linie andere Metall wie z. B. Chrom und Tantal oder Hartstoff-Schichten, vorzugsweise aus Titankarbid, Titannitrid oder Borkarbid; die Beschichtungen haben im allgemeinen Schichtdicken von beispielsweise einigen μm bis zu einigen 1/100 mm. Sie können gegebenenfalls auch aus pyrolytischem Kohlenstoff bestehen.

Das Aufbringen der Schicht auf die Metallfäden erfolgt nach in der Beschichtungstechnik für die Herstellung festhaftender Schichten bekannten Methoden, zu denen beispielsweise — je nach Schichtmaterial — galvanisches Abscheiden oder Aufdampfen, chemische oder physikalische Abscheidung aus der Gasphase, Flamm- oder Plasmaspritzen sowie elektrostatisches Aufspritzen gehören. Beim Aufbringen der Beschichtung ist lediglich darauf zu achten, daß bei dem Beschichtungsverfahren nicht so hohe Temperaturen erreicht werden, daß die mechanischen Eigenschaften des Grundmaterials unzulässig geändert werden. Die Auswahl des geeigneten Grundmaterials, einer geeigneten Beschichtung, der optimalen Schichtdicke und eines geeigneten Vorgehens beim Aufbringen der Schicht erfolgt vorteilhafterweise auf experimentellem Weg.

Die Durchmesser der Metallfäden liegen dabei etwa zwischen 0,01 und 1 mm; ebenso wie bei dem bereits vorgeschlagenen Sehnenersatz ist es möglich, in einer »Sehne« Metallfäden unterschiedlicher Dicke zu verwenden.

Um das Anwachsen von Gewebe an die künstliche Sehne zu fördern, ist es vorteilhaft, wenn der einzelne Faden und/oder mehrere zu einem Strang zusammengefaßte Fäden von einem textilen Geflecht lose umschlossen sind, wobei ein Geflecht aus resorbierbarer Naturseide geeignet ist.

Im folgenden wird die Erfindung anhand eines Ausführungsbeispiels im Zusammenhang mit der Zeichnung näher erläutert.

Fig. 1 zeigt in einem Querschnitt einen aus einzelnen Fäden aufgebauten Strang, der direkt als künstliche Sehne dienen kann;

Fig. 2 ist ein mehrere Stränge der Fig. 1 enthaltendes künstliches Band, in gleicher Darstellung.

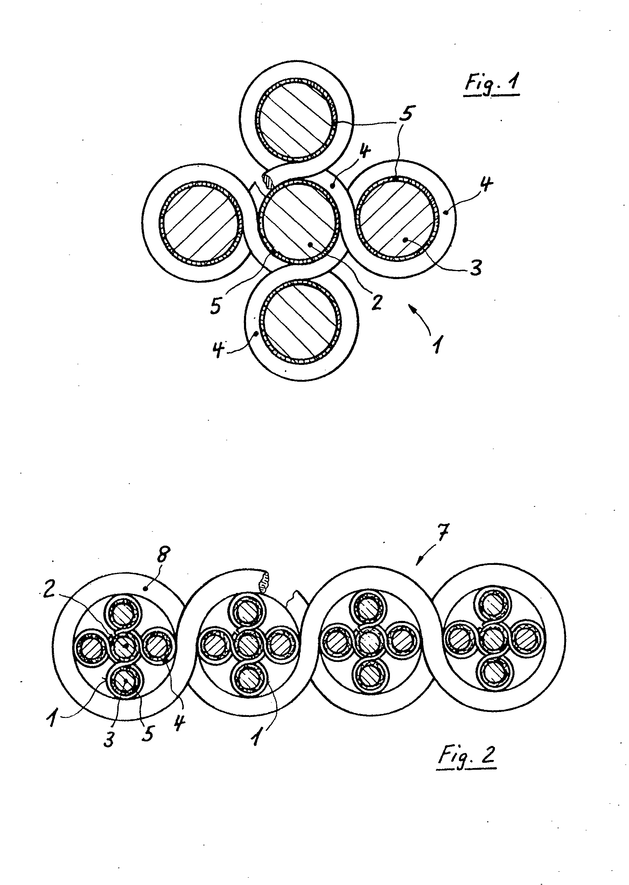

Der in seiner Gesamtheit mit 1 bezeichnete Strang nach Fig. 1 besitzt einen Kernfaden 2 aus einer in der Implantattechnik üblichen Metall-Legierung, beispielsweise rostfreiem Stahl oder CoNiCrMo-Legierung. Um den Kernfaden 2 herum sind in einem Kranz Mantelfäden 3 aus dem gleichen Material konzentrisch angeordnet. Diese Mantelfäden sind — was aus der Zeichnung nicht ersichtlich ist — mit Vorteil leicht seilartig verwunden, d. h. die Mantelfäden 3 laufen in Längsrichtung schraubenförmig um den Kernfaden 2 herum.

Jeder einzelne Faden 2 bzw. 3 ist mit einer Beschichtung 5 aus Ti-Nitrid versehen, die eine Schichtdicke von etwa 1 bis 5 µm hat und mit Hilfe des Verfahrens der chemischen Abscheidung aus der Gasphase aufgebracht worden ist.

Jeder Faden 2 oder 3 sowie der ganze Strang 1 ist von einem textilen Geflecht 4, vorzugsweise aus Naturseide, so umhüllt, daß, wie bereits erwähnt, ein Anwachsen von Körpergewebe an die mechanischen Belastungen aufnehmende Drähte bzw. Fäden 2, 3 erleichtert wird.

Der Durchmesser des Kernfadens 2 kann beispielsweise zwischen 0,1 und 1 mm betragen, während die Mantelfäden 3 Durchmesser zwischen 0,05 und 0,5 mm haben. Die Abstimmung zwischen dem Durchmesser des Kernfadens 2 und demjenigen der ihm im Einzelfall zugeordneten Mantelfäden 3 erfolgt dabei unter Umständen vorteilhafterweise so, daß die Durchmesser der Fäden vom Kern nach außen abnehmen, um die Elastizität des Stranges 1 und damit des daraus gebildeten Bandes von innen nach außen zu steigern.

Obwohl bereits ein Strang 1 als Bänder- oder Sehnenersatz direkt zu verwenden ist, ist es möglich, Stränge 1 als Moduleinheiten für den Aufbau eines Bandes mit höherer Belastungsfähigkeit zu benutzen. Wie in Fig. 2 gezeigt, bilden dabei beispielsweise mehrere Stränge 1 ein flaches Band 7, dessen Form durch ein zweites Geflecht 8, ebenfalls aus Naturseide, gewährleistet wird, das die einzelnen Stränge 1 umschließt.

Eine Verankerung der erfindungsgemäßen Bänder am Knochen kann beispielsweise in der früher beschriebenen Weise erfolgen (EP-A-0 041 111).

Selbstverständlich ist die vorliegende Erfindung nicht auf das gezeigte Ausführungsbeispiel beschränkt, insbesondere nicht hinsichtlich der ausgewählten Materialien und hinsichtlich der erwähnten Verankerung des künstlichen Bandes am Knochen.

## Patentansprüche

1. Sehnen- und/oder Bänderersatz aus einzelnen, in Längsrichtung mindestens annähernd parallel verlaufenden Fäden (2, 3) aus einem körperverträglichen Metall und/oder einer Metalllegierung, die zu mindestens einem Strang (1), bestehend mindestens aus einem Kernfaden (2) und einer Reihe ihn umgebender Mantelfäden (3), zusammengefaßt sind, wobei jeder einzelne Faden (2, 3) mit einer körperverträglichen, korrosionsbeständigen und abriebfesten festhaftenden Beschichtung (5) aus Metall oder einer Hartstoffschicht überzogen ist.

2. Sehnen- und/oder Bänderersatz nach Anspruch 1, dadurch gekennzeichnet, daß der einzelne Faden (2, 3) und/oder mehrere zu einem Strang (1) zusammengefaßte Fäden sowie aus mehreren Strängen (1) aufgebaute Bänder (7) von einem textilen Geflecht (4, 8) lose umschlossen sind.

## Claims

1. An artificial tendon and/or ligament comprising discrete filaments (2, 3) which extend at least substantially parallel to one another in the longitudinal direction and which are made of a biocompatible metal and/or a metal alloy and which are combined to form at least one strand (1) in the form of at least one core filament (2) and a number of outer filaments (3) therearound, each discrete filament (2, 3) having a biocompatible corrosionproof and non-abrading adhering coating (5) of a metal or of a layer of hard material.

2. An artificial tendon and/or ligament according to claim 1, characterised in that the individual filament (2, 3) and/or a number of filaments combined to form a strand (1) and ligemants (7) embodied by a number of strands (1) have disposed loosely around them a textile braiding (4, 8).

## Revendications

1. Produit de remplacement de corde et/ou de bande constitué du fils individuels (2, 3) s'étendant dans le sens longitudinal au moins approximativement en parallèle, en un métal compatible avec le corps et/ou un alliage métallique et qui sont rassemblés pour former au moins un boyau (1), constitué d'au moins un fil (2) formant noyau et d'une série de fils formant enveloppe (3) qui l'entourent, chaque fil individuel (2, 3) étant recouvert d'un revêtement (5), en métal ou en couche de matière dure, compatible avec le corps, résistant à la corrosion, résistant aux frottements et adhérent.

2. Produit de remplacement de corde et/ou de bande selon la revendication 1, caractérisé en ce que le fil individuel (2, 3) et/ou plusieurs fils rassemblés pour former un boyau (1) ainsi que des bandes (7) constituées de plusieurs boyaux (1), sont entourés de façon lâche par un entrelacement textile (4, 8).

Fig. 1

Fig. 2